(19) Europäisches Patentamt
European Patent Office
Office européen des brevets

(11) Publication number: 0 139 484
B1

(12) # EUROPEAN PATENT SPECIFICATION

(45) Date of publication of patent specification: 23.11.89

(51) Int. Cl.$^4$: A 61 F 13/18

(21) Application number: 84306466.8

(22) Date of filing: 21.09.84

(54) **Disposable sanitary napkin product in a flexible shell.**

(30) Priority: 23.09.83 US 535193
29.06.84 US 626167

(43) Date of publication of application:
02.05.85 Bulletin 85/18

(45) Publication of the grant of the patent:
23.11.89 Bulletin 89/47

(84) Designated Contracting States:
AT BE CH DE FR GB IT LI LU NL SE

(56) References cited:
EP-A-0 062 495
AT-B-291 429
DE-A-2 024 831
FR-A-2 521 003
US-A-2 873 740
US-A-3 294 090
US-A-3 344 789
US-A-3 525 337
US-A-3 583 402
US-A-3 805 790
US-A-4 257 418
US-A-4 405 326

(73) Proprietor: PERSONAL PRODUCTS COMPANY
Van Liew Avenue
Milltown New Jersey 08850 (US)

(72) Inventor: Holtman, Dennis C.
Box 125 RD No. 5 Old Clinton Road
Flemington NJ 08822 (US)

(74) Representative: Jones, Alan John et al
CARPMAELS & RANSFORD 43 Bloomsbury Square
London, WC1A 2RA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 139 484 B1

**Description**

The present invention relates to a new and improved disposable sanitary napkin product in a flexible shell having high liquid impact capacity and high liquid retention, and which allows the skin of the wearer to remain dry.

Disposable absorbent products have been known for some time including such products as disposable diapers, sanitary napkins, wound dressings, bandages and incontinence pads. These products incorporate an absorbent batt which is used to absorb and hold or contain body fluids. Initially in many of these products, especially diapers and sanitary napkins, the absorbent batt comprised what is termed "wadding" or plies of tissue. The wadding was disposed between a liquid-impermeable backing and a liquid-permeable facing and the plies of tissue were used to absorb and hopefully contain the liquid within the product. A diaper which utilizes such an absorbent batt is disclosed in US—Re—26,151.

The wadding type of product was replaced for the most part by an improved absorbent batt which comprises what is termed "fluffed wood pulp fibers". This absorbent batt comprises a layer of individualized wood pulp fibers with the layer having substantial thickness. A diaper which incorporates such a fluffed wood pulp absorbent batt is described in US—A—2,788,003. This diaper had improved absorbent capacity and somewhat better containment than a diaper using a wadding layer. Also the fluffed wood pulp layer is quite soft, flexible and conformable, and hence produces an improved diaper or sanitary napkin product over those using wadding as the absorbent layer.

Though the fluffed wood pulp absorbent batts have improved capacity, the efficiency with which the capacity is used in a diaper or sanitary napkin is poor. The reason for this is that the fluid to be absorbed is generally deposited in a localized area within the absorbent batt and the ability of the fluid to move along the plane of the batt is poor. The fluid tends to follow a radial wicking path and consequently moves to the closest edge of the batt where it generally is no longer contained and the product leaks.

US—A—3,017,304 discloses an absorbent product which incorporates in the product a densified paper-like layer. This paper-like layer acts as a wick, i.e. liquid which is placed on the layer tends to move rapidly along the plane of the layer. When incorporated in combination with fluffed wood pulp fiber, the resultant product uses the absorbent capacity of the fluffed wood pulp much more efficiently. Diapers which incorporate this paper-like layer combined with fluffed wood pulp are disclosed and described in US—A—3,612,055 and US—A—3,938,522. This concept of combining wicking ability or a capillary skin or layer with fluffed wood pulp fibers has gained wide acceptance in many absorbent products including disposable diapers and sanitary napkins. Even though these products make much better use of the capacity of the absorbent batt, they still do not totally contain the absorbed liquid. It is probable that these products will leak before the full capacity of the batt is used for the absorption, or at the very least before the entire liquid void by the user is absorbed. This is especially true when pressure is placed on the batt while wet.

When pressure is placed upon a sanitary protection product, the product frequently becomes distorted and menstrual discharge comes in contact with the clothing of the wearer resulting in a stain on the clothing. A recent trend in products in the marketplace is toward thin products. Thus it becomes even more important for the product to retain its shape when placed under stress and pressure. Attempts have been made to provide certain shapes or products to prevent distortion of the product when worn. For example, US—A—3,805,790 provides a sanitary napkin of a particular shape, whereas US—A—4,405,326 uses a molded plastic material of a preformed shape to hold an absorbent material. The thinner napkins and the pre-shaped napkins have still failed to overcome the requirement of rapid absorption and complete retention of discharge and to provide a soft, comfortable product which does not become sufficiently distorted to allow transfer of menstrual fluid stain to clothing.

In another approach to body fluid absorption, a number of years ago "superabsorbent materials", i.e. materials which will absorb many times their weight of liquid were developed. Since the development of such materials, attempts to incorporate them in absorbent products, such as sanitary napkins, to enhance the absorption performance of these products have been made. US—A—4,217,901 suggests placement of a liquid absorbing hydrocolloid material in a particular layer in a pad. Even though superabsorbent materials have been available for some time, they have not gained wide acceptance in absorbent products such as sanitary napkins because of a failure to develop a product capable of economically utilizing the highly increased absorptive capacity of the superabsorbent material. In order to economically utilize the superabsorbent, the liquid being absorbed must be readily accepted and placed in contact with the superabsorbent material. Furthermore, as the superabsorbent material absorbs liquid, it must be allowed to swell. If the superabsorbent is prevented from swelling, it will cease absorbing liquid. Hence, if the superabsorbent material is to function in sanitary napkin products, wherein the liquid to be absorbed is placed in a small void area, the structure of the absorbent layer containing superabsorbent materials must have certain characteristics.

The present invention provides a new and improved sanitary protection product which possesses a large storage capacity, is soft and comfortable, and can be designed so as not to be apparent through normal clothing. The product

can also utilize a substantial portion of the absorptive capacity of superabsorbent materials. In addition, the new absorbent product will contain the absorbed liquid and not transfer it to apparel of the wearer, even when pressure is placed upon the product during use.

AT—B—0 291 429 discloses an absorbent product comprising a shell containing an absorbent material. The shell may comprise a plastics foam material, such as polyurethane, polyethylene, polystyrene or polyvinylchloride foam.

US—A—4 257 418 discloses a holder for multiple use adapted to contain a disposable diaper to form an incontinence pad. The holder is generally boat shaped.

EP—A—0 062 495 discloses an absorbent pad comprising an undulating, unsecured batt made from a sheet of a fibrous absorbent having a water impermeable sheet on one face and being enclosed in a water-permeable wrap.

DE—A—2 024 831 discloses an absorbent urinary pad comprising a shell and a superstructure within the shell. The superstructure comprises polyester fibers in intimate contact with an absorbent medium.

EP—A—0 140 470, which is prior art for novelty purposes only by virtue of Article 54(3) EPC discloses a disposable urinary pad comprising: a liquid-impermeable substantially flexible shell having a depth of at least 0.5 inch (1.27 cm); a superstructure placed in and substantially filling said shell, said superstructure being at least slightly compressible and capable of maintaining a void volume of liquid, said superstructure being selected from the group consisting of a fibrous web, a foam, entangled resilient fibers, and mixtures thereof; and an absorbent medium in intimate contact with at least a portion of said superstructure.

US—A—3 525 337 discloses an absorbent element for sanitary napkins or the like consisting of an accordion-pleated pad formed from a thin layer of absorbent fibers faced on each side with sheets of absorbent cellulose wadding. The pleats are secured in their folded configuration by having the peaks of the folds on one side of the pad attached to an anchoring sheet.

US—A—3 805 790 discloses a disposable sanitary protection product comprising:

a liquid impermeable, substantiallly flexible, moulded shell;

a fibrous superstructure, placed in and substantially filling said shell, which is at least slightly compressible and capable of retaining a void volume of liquid; and

an absorbent medium in intimate contact with at least a portion of said superstructure.

The present invention provides a disposable sanitary protection product which is characterised in that:

the shell had a depth, measured from a line extending across the width at the shell rim in the central portion, of from 0.125 to 0.5 inch (0.3175 to 1.27 cm); and

the superstructure comprises a corrugated fibrous web fibrous having a wet bulk of at least 30 cc/g and stabilized to retain its corrugations when wet by face-to-face bonding between corrugations.

The shell is preferably a polyethylene foam shell which is formed from a blown polyethylene foam sheet and subsequently subjected to molding by a thermal process. The shell preferably has a boat-like shape and ranges in thickness from 1/64 inch (0.4 mm) to 1/4 inch (6.4 mm). The shell has a length which preferably ranges from 4 inches (10.2 cm) to 8 inch (20.3 cm), and a width measured from one rim to another across the top space preferably from 2 inches (5.1 cm) to 5 inches (12.7 cm).

Preferably, the superstructure comprises a fibrous nonwoven web formed of a resilient fiber such as polyester. The web is corrugated and stabilized to prevent the corrugations from separating or flattening when the web is wet and has pressure placed upon it.

The absorbent medium is for example a superabsorbent material, hydrophilic fibers which are loosely compacted or formed into a nonwoven web, wadding, tissue, peat moss or mixtures thereof.

Preferably, a liquid-permeable fabric or web covers the side of the shell which is open. This cover or facing may be sealed to the rim of the shell thereby entrapping the superstructure and the absorbent medium which has been placed in the shell. If the pad does not have a facing or covering, the superstructure and, if necessary, the absorbent medium, are affixed to the shell so as to remain in position even when wet.

The product of the present invention has a high impact capacity, i.e. the product receives a relatively large quantity of liquid and retains it. Furthermore, the product does not leak or spill over. In other words, once the body exudate enters the pad, it remains entrapped within the product. The product also has a high liquid-holding capacity. In addition, the product maintains its surface dry, thereby keeping any moisture away from the skin of the wearer and prevents any contact with clothing so as to avoid staining. Still further, the product of the present invention permits air circulation in the region where the product is worn which results in a high degree of comfort and assists in providing for odour control.

Some embodiments of the present invention are now described by way of example only, with reference to the accompanying drawings, in which:

Figure 1 is a perspective view of one embodiment of the present invention with a portion broken away for further clarification.

Figure 2 is a perspective view of the parts prior to assembly which provides one embodiment of the present invention;

Figure 3 is a cross-sectional view taken through lines 3—3 of Figure 2; and

Figure 4 is a cross-sectional view like Figure 3 of still another embodiment of the invention.

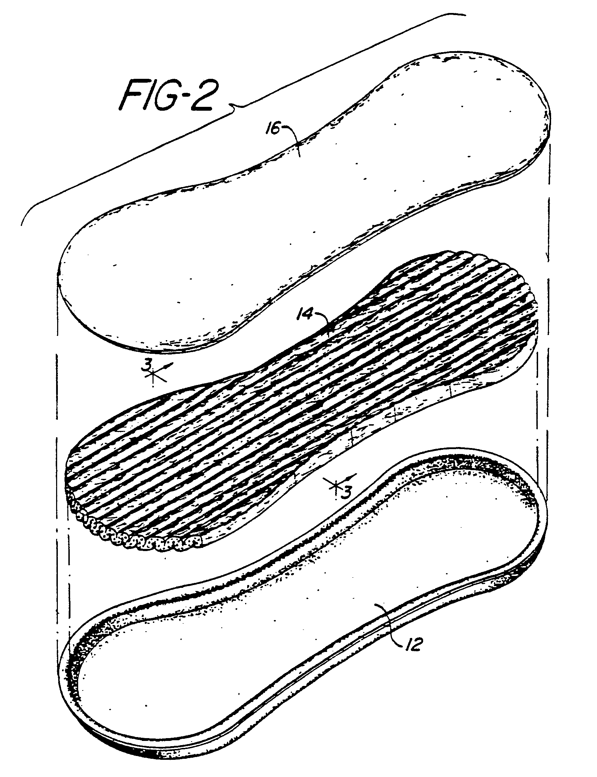

Figure 1 depicts a sanitary protection product 10 having a shell 12 with a depth of 0.25 inch (0.635 cm). The shell 12 contains a corrugated fibrous web 14 and has a facing 16 sealed to the rim of the shell 12.

Figure 2 illustrates the same sanitary protection product 10 from Figure 1 with the elements separated and showing their relationship. The shell 12 is a polyethylene foam shell which is preformed by a thermal molding process known in the art. The shell is 0.0625 inch (0.16 cm) thick and is boat shaped. The top of the shell rims are at least 0.125 inch (0.3175 cm) in depth from the bottom most point of the shell. On the underside of the shell 12 are adhesive lines which are applied to provide the securement means for securing the sanitary protection product to the clothing of the user. These adhesive lines are covered with release strips which, when peeled from the adhesive strips leave the adhesive tacky. The superstructure 14 is a corrugated fibrous web which generally is at least 0.125 inch (0.3175 cm) thick and contains about 4 or 5 corrugations per inch (2.5 4 cm). In a specific embodiment, a polyester fibrous web is carded, which web has a basis weight of about 25 grams per square meter. The web is corrugated, in other words transversely folded, by known procedures such as that exemplified in US—A—4,111,733. The web is stabilized by face-to-face bonding between corrugations so that when the web becomes wet, it does not lose its corrugated configuration. In this embodiment the absorbent medium in the form of superabsorbent (not shown) is affixed to the corrugated web as small particles or film-like partial coverings of the web fibers. The facing 16 is a liquid-permeable, generally hydrophobic fibrous web which may have a typical basis weight of 0.5 oz/yd$^2$ (16.95 g/m$^2$). The three elements, the shell 12, the absorbent structure 14 and the facing 16, are combined as shown in the drawing, the facing being sealed at its edge to the rim of the shell so as to provide a unitary product.

Figure 3 is a cross-sectional view along line 3—3 of Figure 2, showing a portion of a typical corrugated web. This portion 30 of the web shows the web 34 in a corrugated form wherein superabsorbent 32 has been placed among the fibers of the web. The web has been stabilized by thermal bonding of fusible fibers 36 which are in the blend of fibers forming the web 34.

Figure 4 is a cross-sectional view of still another portion 50 of a corrugated web 52. This corrugated web 52 contains two layers 54 and 56. The layer 54 is a fibrous layer which has a lower capillary pressure than the second layer 56. The corrugated web 52 is stabilized by fusible fibers 58. When the web is exposed to a temperature which substantially melts these fibers the corrugations in the web are partially fused together.

These and other products such as other sanitary protection products may be made along the same lines as the products depicted in Figures 1—4.

The liquid-impermeable subtantially flexible shell is formed from a moldable substance. The substance when molded should provide a liquid-impermeable substantially flexible shell with a thickness ranging from 0.015625 to 0.25 inch (0.04 to 0.635 cm). The shell after deformation should substantially return to its original shape.

Substances which provide these characteristics and which are moldable by pressure or thermal molding or the like are suitable. Particularly suitable for use in the present invention is a polyethylene-containing foam.

The polyethylene-containing foam shell is prepared by known thermal molding processing. The preferred formulation for forming the polyethylene-containing foam material is identified as Volara®, which is a polyethylene ethylvinyl acetate blend. The product is manufactured and sold by Voltek, Inc., Lawerence, Massachusetts. Preferably, the formulation is prepared in sheet form at approximately 0.0625 to 0.125 inch (0.16 to 0.3175 cm) in thickness. The sheet is subjected to thermal molding at a temperature of about 260°F (127°C) to form the foam shell. The shell is boat-like in shape, but is not limited thereto. The depth of the shell is measured by extending a line horizontally from one rim to another in the center of the product perpendicular to the longitudinal axis of the product. The depth is then measured from that line to the base of the foam shell on the longitudinal axis. This depth ranges from 0.125 to 0.5 inch (0.3175 to 1.27 cm). The foam shell may be made of other suitable compositions which are soft and flexible and are liquid impermeable.

The superstructure may be comprised of one or more fibrous webs, possibly mixed with resilient fibers which are entangled. A fibrous web generally is formed from synthetic fibers such as polyethylene, polypropylene, polyester, polyamide fibers, bicomponent fibers, copolymers thereof or mixtures thereof. However, cellulosic fibers such as rayon may be used.

The fibers are placed in the web by known means such as by carding to form a web which is then stabilized by face-to-face bonding between corrugations.

Stabilization may be achieved by heat-through bonding, adhesive bonding, point embossing with heat or adhesive or both, needle punching or use of water jets. The stabilizing process is selected according to the fibers used and the process used to form the web. Other suitable procedures for forming a web include air-laying, wet-laying, spun bonding, laying of melt blown fibers, spread tow and other known techniques. A typically suitable web for a sanitary protection product has a dry bulk of at least 10 cc/gram, a weight less than 4 oz/yd$^2$ (135.6 g/m$^2$) and a wet bulk of at least 30 cc/gram.

The fibrous web is corrugated and stabilized so as to prevent loss of corrugation when the fibrous web becomes wet. Corrugating or transverse folding of the web may be carried out by procedures such as that in US—A—4,111,733. Generally the web corrugations will range from about 3 to 6 or even 8 per inch (2.54 cm) of

corrugated web and the web thickness will be from 0.125 to 0.5 inch (0.3175 to 1.27 cm) in thickness. One method of stabilizing the corrugations is accomplished by using an adhesive which may be a latex binder or other known adhesive. Another method of stabilizing the web is by adding a small portion of fusible fibers to the web fibers before or after the web is made. These fusible fibers have a lower melting point than the remaining fibers and when the corrugated web is subjected to temperatures sufficient to melt the fusible fibers, light bonding is provided between the corrugations.

In one specific embodiment, a blend of staple polyester fibers with a minor portion of fusible fibers such as lower melt polyester fibers are carded to form a web. The web is subsequently lightly bonded by passing hot air through the fibers making the fusible fibers tacky so as to stick to each other and the staple fibers to provide the desired degree of integrity to the corrugated web structure.

If the desired thickness is not available in the web, more than one layer of the web may be used but preferably the capillary pressure provided by each web layer increases as the layers are placed away from the facing. In an example with three layers of fibrous webs, the top layer, that is the layer closest to the open side of the shell, has the lowest capillary pressure, the mid layer has a higher capillary pressure than the first layer but a lower capillary pressure than the third and last layer.

What appears to be only a small difference in capillary pressure is all that is required for one layer to attract and drain liquid from an adjacent layer. The force causing a liquid to enter a cylindrical capillary is expressed by the equation:

$$P=\frac{(2v \cos \theta)}{r}$$

wherein the force is represented by the capillary pressure and

P is the capillary pressure,

v is the surface tension of the liquid,

θ is the liquid-fiber contact angle, and

r is the capillary radius.

With a given liquid, the pressure (capillary force) increases with the cosine of the liquid-fiber contact angle (reaching a maximum where the angle is zero) and also increases with narrower capillary radii so that narrower capillaries will draw liquid from wider ones.

The relative wickability between a first fibrous layer and a second layer is affected by both the relative densities of the layer and the relative wetability of the individual fibers in each layer. The individual fibers of the second layer preferably have substantially smaller liquid fiber contact angles than those of the first fibrous layer overcoming the density difference and providing a significant overall increase in capillary pressure to absorb liquid into the second layer.

The fibers of the second layer of fibers and any subsequent layer of fibers (or particles) and/or the density of the layer, are selected to create a significant difference in capillary pressure from the first fibrous layer.

The second fibrous (or particle) layer is generally comprised of fibers having a lower liquid-contact angle or wherein the layer is provided with a narrower capillary radii. Examples of such fibers include hydrophilic fibers such as rayon fibers, cellulosic fibers, or peat moss, or mixtures thereof, or acrylic fibers. Cellulosic fibers include wood pulp fibers and cotton linters.

The wood pulp fibers generally are those that are used to form the fluff or fibrous batt layer in conventional absorbent products such as sanitary napkins. Other cellulosic fibers that might be used are rayon fibers, flax, hemp, jute, ramie and cotton. The fiber, or peat moss, or mixtures thereof are placed in such a way as to form a layer in which the particles are close to one another so as to promote wicking of liquid in the plane of the layer.

The second layer can be preformed and placed next to the first fibrous layer or the particles (fibers or peat moss or mixtures thereof) can be air-laid or wet-laid or otherwise combined with the first fibrous layer before any transverse folding or corrugating takes place.

The multiple layer structure is then corrugated.

Entangled fibers may be used to provide the superstructure. These fibers should be resilient and have sufficient denier (tex) to provide the adequate void volume within the foam shell. The fibers may be frictionally entangled or otherwise entangled so as to provide the necessary void volume. Typical of fibers suitable for use are synthetic fibers such as polyethylene, polypropylene, polyester, nylon, bicomponent fibers, copolymers thereof, or mixtures thereof, or cellulosic fibers such as rayon fibers, and acrylic fibers.

The superstructure is selected so as to provide sufficient void volume to hold a normal liquid void and impact capacity to receive the liquid rapidly enough to prevent a run off.

The superstructure has a wet bulk of at least 30 cc/gram. The wet bulk is the area times thickness of the layer under a load 0.01 psi (69 Pa) calculated in cubic centimeters when the layer has been saturated with water. This value is divided by the dry weight in grams of the fibrous layer in order to provide the measurement in cubic centimeters per gram. The load 0.01 psi (69 Pa) is placed on the web during testing in order to confine the surface fibers to the fibrous layer so as not to cause any false calculation due to fibers projecting beyond the fibrous layer. The superstructure should retain liquid even under normal pressure such as that provided by the wearer of the product when sitting down or moving the legs thereby compressing the sanitary product.

Generally the superstructure will substantially fill the shell. However, it is necessary to fill the shell only to the extent required to hold the void during use of the product.

In addition to the fibrous webs discussed above

of wood pulp fibers and the like, the absorbent medium can be comprised of superabsorbent which is placed in the manners discussed hereinbefore in the product. Superabsorbent provides an increase in the liquid capacity of the product and is placed in intimate contact with at least a portion of the superstructure.

The superabsorbent, present either on the fibers or placed in the folds of a corrugated web, or otherwise associated with the void volume portion of the superstructure, is generally a water-insoluble, water-swellable polymeric substance capable of absorbing water in an amount which is at least 10 times the weight of the substance in its dry form. The superabsorbent is in the form of fibers, spheres, particles, bits of film, globules, webs or film, or may be applied in the form of a liquid monomer solution which is subsequently polymerized. The superabsorbent prepared by polymerization of a monomer solution placed on fibers in a web is most frequently in the form of globules and bits of film-like particles in the web structure.

One type of superabsorbent material provides particles or fibers which may be described chemically as having a backbone of natural or synthetic polymers with hydrophilic groups or polymers containing hydrophilic groups being chemically bonded to the backbone or an intimate mixture therewith. Included in this class of materials are such modified natural and regenerated polymers as polysaccharides, including for example, cellulose and starch and regenerated cellulose which are modified by being carboxyalkylated, phosphonoalkylated, sulfoalkylated, or phosphorylated to render them highly hydrophilic. Such modified polymers may also be crosslinked to improve their water-insolubility.

These same polysaccharides may also serve, for example, as the backbone on to which other polymer moieties may be bonded by graft copolymerization techniques. Such grafted polysaccharides and their method of manufacture are described in US—A—4,105,033 and may be described as polysaccharide chains having grafted thereon a hydrophilic chain of the general formula:

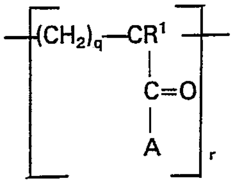
·
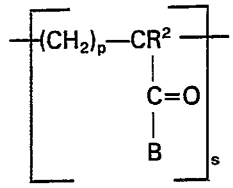

wherein A and B are selected from the group consisting of $—OR^3$, —O(alkali metal), $—OHNH_3$, $—NH_2$, wherein $R^1$, $R^2$, and $R^3$ are selected from the group consisting of hydrogen and alkylene having 1 to 4 or more carbon atoms, wherein r is an integer having a value of 0 to about 5000 or more, s is an integer having a value of 0 to about 5000 or more, r plus s is at least 500, p is an integer having a value of 0 or 1, and q is an integer having a value of 1 to 4. The preferred hydrophilic chains are hydrolyzed polyacrylonitrile chains and copolymers of polyacrylamide and polysodium acrylate.

In addition to the modified natural and regenerated polymers, the hydrocolloid component may comprise wholly synthetic hydrophilic particles. Examples of those now known in the art are polyacrylonitrile fibers which may be modified by grafting moieties thereon such as polyvinylalcohol chains, polyvinyl alcohol itself, hydrophilic polyurethane, poly(alkyl phosphonates), partially hydrolyzed polyacrylamides (e.g. poly(N,N-dimethylacrylamide), sulfonated polystyrene, or a class of poly(alkyleneoxide). These highly hydrophilic synthetic polymers may be modified by other chemical treatments such as crosslinking or hydrolysis. Further examples known in the art are the non-ionic polymers such as polyoxyethylene, polyoxypropylene, and mixtures thereof which have been suitably crosslinked, either chemically or by irradiation. Still another more recent type is a derivative of isobutylene-malic and acrylate monomers, such as sodium, potassium, ammonium, (or a combination of cations), acrylate, may be placed on the absorbing layer by spraying or otherwise placing a solution thereon, followed by polymerization and crosslinking, for example, by irradiation.

In addition, naturally occurring materials such as gums may be used. Examples of such suitable gums include guar gums, acacia gums and locust bean gums.

The superabsorbent may be placed in the bottom of the shell prior to the placing of the superstructure in the shell or it may be a part of the superstructure. If the superstructure is a fibrous web having substantially uniform density throughout, the superabsorbent is best placed between the fibrous web and the inside surface of the shell. Another alternative method of placing superabsorbent on or within a fibrous web, is by spraying a monomer solution on the fibrous web or perhaps even saturating the web with a monomer solution followed by polymerization and crosslinking of the monomer. One typical way to polymerize the monomer is by use of irradiation. This places the superabsorbent substantially evenly throughout the fibrous web and affixes the superabsorbent in such a manner that the superabsorbent globules or particles are within a void volume sufficient to permit them to swell substantially to completion.

The superabsorbent can be placed within the folds provided it is sufficiently associated with the void volume that the swelling of the superabsorbent can occur. If the web is a multiple layer web, it is desirable to associate the superabsorbent with the web having the highest capillary pressure. Another concept is the placement of superabsorbent in a pre-established situation, such as within a moisture-permeable bag such as a "T-bag", or a pocket. If the superabsorbent is in the form of granules, it may be desirable to moisten the granules and then fix them in place either on the web or at the surface of the shell, which will be in contact with the superstructure.

In summation, the superstructure has at least a slight degree of compressibility, allows liquid to enter the structure rapidly, retains the liquid, and provides collapse resistance so that the liquid is not pressed out of the superstructure.

The product of the present invention does not require a facing or cover but if no facing or covering is used, then it is necessary to secure the superstructure within the shell so that prior to or during use the superstructure does not separate itself from the shell. If, however, it is desirable to use a covering or facing, the covering or facing placed over the open side of the shell is liquid-permeable and is readily sealable to the outer rim of the shell so as to entrap the superstructure in the shell. Suitable coverings or facings include fabrics, nonwoven webs and perforated films. Preferably, the facing is a thermoplastic substance which can be heat sealed to the rim of the liquid-impermeable shell.

The product of the present invention is worn by the wearer in the crotch region, and for simplicity is secured to the underclothing of the wearer. Securement may be effected by adhesive lines or strips on the exterior of the shell or may simply secure itself to the underclothing by means of friction. If the product is to be secured by friction, a material for manufacturing the shell is selected which will provide sufficient friction or a material is coated on the exterior of the shell to provide such friction.

Examples for the preparation of embodiments of the present invention are as follows. These examples are not intended to be limiting in any way and extensions and modifications thereof, without departure from the scope of the invention, will become apparent from these examples.

Example I

A shell is formed similar in design to that depicted in Figure 1 for a sanitary protection product. The shell is thermoformed from a foam sheet which is a blend of polyethylene and ethylvinyl acetate. The shell has a length 7.75 inch (19.7 cm) and a width at the widest point of 3 inch (7.6 cm) and a width at the narrowest point of 2.25 inch (5.7 cm).

The shell is 0.5 inch (1.27 cm) deep at the center from a line extending across the center from the edge of each rim of the shell.

The superstructure placed in the shell consists of a two layer fibrous web which has been corrugated. The upper layer which consists of 50 percent by weight of the web is a blend of 75 parts of polyester fibers with 25 parts of polyester binder fibers. The average denier (tex) of the fibers is about 15 (1.7). The second layer also making up 50 percent of the web is 40 parts orlon® fibers of 1.5 denier (0.17 tex) blended with 10 parts by weight of the same binder fibers used in the top layer. Each of the layers of the web are carded and one layer is placed upon the other. The two layer web structure is then corrugated and heat set at 315°F (157°C). The corrugated two layer web is slightly less than 0.5 inch (1.27 cm) high and has approximately 4.5 folds per inch (2.54 cm) of corrugation. The corrugated web has a weight of about 7 oz/yd$^2$ (237 g/m$^2$).

The absorbent medium is a blend of superabsorbent identified as 10SH manufactured and sold by Mitsubishi Company, Tokyo, Japan, and mineral oil. 4 parts of superabsorbent are mixed with one part of mineral oil. This blend is placed on the bottom fiber layer of the two layer web and between the folds to a depth of approximately 0.125 to 0.25 inch (0.3175 to 0.635 cm). The amount of the blend added is approximately one gram of superabsorbent mineral oil blend per gram of superstructure.

A nonwoven fabric made from bicomponent fibers of polyester core and polyethylene sheath having a weight of 0.5 oz/yd$^2$ (16.95 g/m$^2$) is heat sealed to the rim of the shell to provide a facing or covering for the product.

The product when worn absorbs menstrual fluid readily and does not distort or stain clothing of the wearer.

It becomes readily apparent from the above example that the present invention provides a highly satisfactory product for those requiring sanitary protection products.

**Claims**

1. A disposable sanitary protection product comprising:

a liquid impermeable, substantially flexible, moulded shell (12);

a fibrous superstructure (14), placed in and substantially filling said shell (12), which is at least slightly compressible and capable of retaining a void volume of liquid; and

an absorbent medium (32) in intimate contact with at least a portion of said superstructure

characterised in that:

the shell (12) had a depth, measured from a line extending across the width at the shell rim in the central portion, of from 0.125 to 0.5 inch (0.3175 to 1.27 cm); and

the superstructure (14) comprises a corrugated fibrous web having a wet bulk of at least 30 cc/g and being stabilized to retain its corrugations when wet by face-to-face bonding between corrugations.

2. The product of claim 1, wherein the shell (12) has a thickness of from 0.016 to 0.25 inch (0.04 to 0.635 cm).

3. The product of claim 1 or claim 2, wherein the shell (12) comprises a thermoformable substance.

4. The product of any one of claims 1 to 3, wherein the shell (12) has a boat-like shape.

5. The product of any one of claims 1 to 4, wherein the corrugated fibrous web is a polyester web.

6. The product of any one of claims 1 to 5, wherein the absorbent medium is loosely compacted wood pulp fibers.

7. The product of any one of claims 1 to 5, wherein the absorbent medium is a superabsorbent material.

8. The product of any one of claims 1 to 7, wherein said corrugated web is face-to-face bonded by adhesive.

9. The product of any one of claims 1 to 7, wherein said corrugated web is face-to-face bonded by thermal bonding of heat fusible fibers contained in the web.

**Patentansprüche**

1. Wegwerfdamenbinde, umfassend

— eine flüssigkeitsundurchlässige, im wesentlichen biegsame geformte Schale (12);

— einen faserigen Aufbau (14), der in der Schale (12) angeordnet ist und dieselbe im wesentlichen ausfüllt, wobei der Aufbau wenigstens geringfügig zusammendrückbar und befähigt ist, ein ausgeschiedenes Flüssigkeitsvolumen zurückzuhalten; und

— ein absorbierendes Medium (32), das sich in inniger Berührung mit wenigstens einem Teil des Aufbaues befindet;

dadurch gekennzeichnet, daß

die Schale (12) eine Tiefe von 0,125 bis 0,5 Inch (0,3175 bis 1,27 cm), gemessen von einer Linie aus, die sich quer über die Breitenerstreckung am Schalenrand im Mittelteil erstreckt, aufweist;

und daß der Aufbau (14) eine gewellte Faserbahn umfaßt, welche ein spezifisches Naßvolumen von wenigstens 30 $cm^3/g$ aufweist und durch direkte Bindung zwischen den Wellungen stabilisiert ist, um ihre Wellungen im nassen Zustand beizubehalten.

2. Produkt nach Anspruch 1, worin die Schale (12) eine Dicke von 0,016 bis 0,25 Inch (0,04 bis 0,635 cm) hat.

3. Produkt nach Anspruch 1 oder 2, worin die Schale (12) eine unter Wärmeeinwirkung verformbare Substanz umfaßt.

4. Produkt nach einem der Ansprüche 1 bis 3, worin die Schale (12) eine bootähnliche Form hat.

5. Produkt nach einem der Ansprüche 1 bis 4, worin die gewellte Faserbahn eine Polyesterbahn ist.

6. Produkt nach einem der Ansprüche 1 bis 5, worin das absorbierende Medium aus locker verdichteten Holzzellstoffasern besteht.

7. Produkt nach einem der Ansprüche 1 bis 5, worin das absorbierende Medium ein superabsorbierendes Material ist.

8. Produkt nach einem der Ansprüche 1 bis 7, worin die gewellte Bahn direkte Klebstoffbindungen aufweist.

9. Produkt nach einem der Ansprüche 1 bis 7, worin die gewellte Bahn auf Grund des thermischen Bindens von warmverschweißbaren Fasern, die in der Bahn enthalten sind, direkt gebunden ist.

**Revendications**

1. Un produit de type protection hygiénique jetable comprenant:

une enveloppe moulée (12) imperméable aux liquides susbtantiellement souple;

une superstructure fibreuse (14) placée dans et remplissant substantiellement ladite enveloppe (12) qui est au moins légèrement compressible et capable de retenir un volume de liquide d'évacuation; et

un milieu absorbant (32) en contact étroit avec au moins une partie de ladite superstructure

caractérisé en ce que:

l'enveloppe (12) a une profondeur mesurée à partir d'une ligne s'étendant le long de la largeur au niveau du bord de l'enveloppe dans la partie centrale allant de 0,125 à 0,5 pouces (0,3175 à 1,27 cm); et

la superstructure (14) comprend un tissu fibreux plissé ayant un volume mouillé d'au moins 30 $cm^3/g$ et étant stabilisé par liaison face à face entre les plis pour retenir ses plis lorsqu'il est mouillé.

2. Le produit selon la revendication 1 dans lequel l'enveloppe (12) a une épaisseur allant de 0,016 à 0,25 pouces (0,04 à 0,635 cm).

3. Le produit selon la revendication 1 ou 2 dans lequel l'enveloppe (12) comprend une substance thermoformable.

4. Le produit selon l'une des revendications 1 à 3 dans lequel l'enveloppe (12) a une forme semblable à un bateau.

5. Le produit selon l'une des revendications 1 à 4 dans lequel le tissu fibreux plissé est un tissu de polyester.

6. Le produit selon l'une des revendications 1 à 5 dans lequel le milieu absorbant est des fibres de pulpe de bois lâchement resserrées.

7. Le produit selon l'une des revendications 1 à 5 dans lequel le milieu absorbant est un matériau superabsorbant.

8. Le produit selon l'une des revendications 1 à 7 dans lequel ledit tissu plissé est lié face à face par de l'adhésif.

9. Le production selon l'une des revendications 1 à 7 dans lequel ledit tissu plissé est lié face à face par liaison thermique de fibres fusibles à la chaleur contenues dans le tissu.

FIG-1

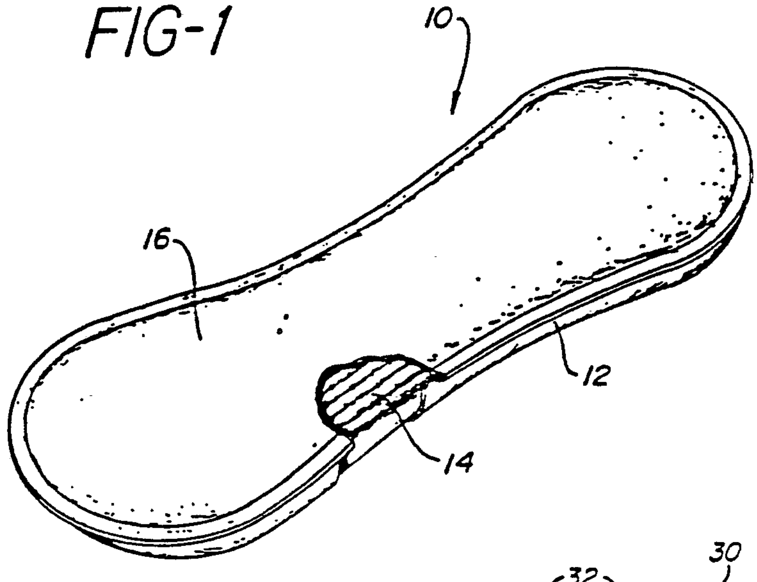


FIG-3

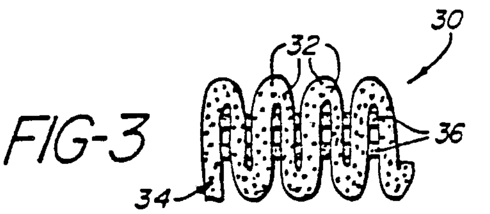


FIG-4

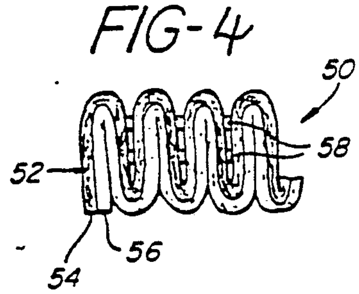

FIG-2